# EUROPEAN PATENT APPLICATION

(11) **EP 4 512 459 A1**
(43) Date of publication of application: **26.02.2025**
(21) Application number: 23810981.3
(22) Date of filing: 22.05.2023
(51) Int. Cl.: A61M 60/232, A61M 60/419, A61M 60/492, A61M 60/804, A61M 60/814

(54) **MAGNETIC LEVITATION CENTRIFUGAL PUMP**

(30) Priority: 23.05.2022 CN 202210565543
(71) Applicant: RocketHeart Technology Co. Ltd, Tianjin 300457 (CN)
(72) Inventor: HAN, Zhifu, Tianjin 300457 (CN); WU, Wenjin, Tianjin 300457 (CN); FAN, Qinglin, Tianjin 300457 (CN); ZHANG, Xuman, Tianjin 300457 (CN); DAI, Ying, Tianjin 300457 (CN); JING, Pengfei, Tianjin 300457 (CN); SONG, Guogang, Tianjin 300457 (CN)
(74) Representative: Zacco GmbH
(86) International application number: PCT/CN2023/095432
(87) International publication number: WO 2023/226910

(57) **Abstract**

Disclosed is a magnetic levitation centrifugal pump, including a volute (200), a static magnetic ring (22) and a rotor (100). The volute (200) is provided with a levitation cavity, a medium inlet and a medium outlet; the rotor (100) is located inside the levitation cavity, and the static magnetic ring (22) is fixed to the volute; the rotor (100) includes a rotor body, a dynamic magnetic ring (16) positioned on the rotor body and at least two blades (13); the dynamic magnetic ring (16) is coaxial and nested with the static magnetic ring (22) to limit the radial position of the rotor body relative to the volute (200); the rotor body is fixedly provided with a magnet steel assembly (14); the rotor body is also fixedly provided with a magnetic member (17); the volute (200) is packaged with a driving coil assembly (21) arranged opposite the magnet steel assembly (14), and the driving coil assembly (21) cooperates with the magnet steel assembly (14) to cause the rotor body to rotate in the circumferential direction; and the volute (200) is fixedly provided with a magnetic levitation coil assembly (27), and when the magnetic levitation coil assembly (27) is energized, the magnetic member (17) and the magnetic levitation coil assembly (27) generate an axial force. By the magnetic interaction between the dynamic magnetic ring (16) and the static magnetic ring (22), the full levitation operation of the rotor (100) can be achieved, thereby reducing heat generation and wear, and maximally reducing the possibility of thrombus generation and crushing damage to blood cells. The rotational driving and axial position control of the rotor (100) are completely independent, and the control logic is simple.

## Description

The present application claims the priority based on Chinese Patent Application No. 202210565543.2, filed with the Chinese Patent Office on May 23, 2012 and entitled "Magnetic Levitation Centrifugal Pump", which is incorporated herein by reference in its entirety.

### Technical Field

The present invention relates to the field of vibration reduction technology, and in particular, to a magnetic levitation centrifugal pump.

### Background

Heart failure (commonly abbreviated as " 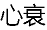 " in Chinese), refers to the condition where a natural heart is unable to pump sufficient blood to maintain blood circulation throughout the body. According to statistics from the World Health Organization (WHO), approximately 15% to 20% of people suffer from varying degrees of heart failure. Over 50% of hospitalizations for individuals aged 65 and older are due to heart failure, and the mortality rate exceeds 50% within 5 years. For patients with heart failure, there are only three treatment approaches: conservative drug therapy, heart transplantation, and ventricular assist devices. The effect of drug therapy is poor, and heart transplantation is very difficult due to the limitation of donors, and therefore, ventricular assist devices (VAD) are recognized as the most effective treatment approach for various types of end-stage heart failure worldwide. The main component of a ventricular assist device is a blood pump (in English). Generally, an inflow pipe of a blood pump is connected with a left ventricle or a right ventricle of a human heart, while an outflow pipe is connected with an aorta or a pulmonary artery; the pump is connected with a control driver (with a power supply device); and the control driver controls the blood pump to output blood at a specific pressure (generally ranging from 80 to 120 mmHg) and flow rate (generally ranging from 2 to 10L/min), thereby assisting in meeting the normal power demands of the human heart during regular activities of the body.

In view of the limitations of the use environment of the blood pump,, how to achieve high integration and compact size characteristics for blood pumps while meeting functional requirements is a key technical issue always concerned by those skilled in the art.

### Summary

The purpose of the present invention is to provide a magnetic levitation centrifugal pump which has a small volume and a compact structure.

The present invention provides a magnetic levitation centrifugal pump, including a volute, a static magnetic ring and a rotor;
the volute is provided with a levitation cavity, a medium inlet and a medium outlet, the rotor is located inside the levitation cavity, and the static magnetic ring is fixed to the volute;
the rotor includes a rotor body, a dynamic magnetic ring positioned on the rotor body, and at least two blades; the dynamic magnetic ring is coaxial and nested with the static magnetic ring to limit a radial position of the rotor body relative to the volute;
the rotor body is fixedly provided with a magnet steel assembly, the magnet steel assembly includes N first magnet steels arranged in the circumferential direction, and magnetic poles of all the first magnet steels are arranged in an alternating manner; the rotor body is further fixedly provided with a magnetic member, and the magnetic member includes at least one of a magnetic ring and an iron core;
the volute is encapsulated with a driving coil assembly arranged opposite the magnet steel assembly, and the driving coil assembly cooperates with the magnet steel assembly to enable the rotor body to rotate in the circumferential direction; and the volute is fixedly provided with a magnetic levitation coil assembly, and when the magnetic levitation coil assembly is energized, the magnetic member and the magnetic levitation coil assembly generate an axial force.

In some embodiments, the magnetic levitation centrifugal pump further includes a position sensor for detecting an axial position of the rotor body;
or/and adjacent first magnet steels are attached, or the magnet steel assembly further includes transverse magnetic conductive steels, each of the magnetic conductive steels is located between two of the first magnet steels, and all the magnetic conductive steels and all the first magnet steels form a Halbach magnet steel array.

In some embodiments, the magnet steel assembly and the magnetic member are respectively provided on a first end portion and a second end portion of the rotor body, and the driving coil assembly and the magnetic levitation coil assembly are respectively provided on a first end portion and a second end portion of the volute.

In some embodiments, an inner cavity of the volute is provided with a first annular housing and a second annular housing; the first annular housing and the second annular housing are respectively located on a first end portion and a second end portion of the volute; the first annular housing and the volute enclose a first sealed cavity for encapsulating the driving coil assembly ; the second annular housing and the volute enclose a second sealed cavity for encapsulating the magnetic levitation coil assembly; the levitation cavity is formed between the first annular housing and the second annular housing; the first annular housing and the second annular housing are both of a ceramic structure; the driving coil assembly is attached to the first annular housing; and the magnetic levitation coil is attached to the second annular housing.

In some embodiments, the rotor body includes an annular body and a base body, wherein the annular body and the base body are fixedly arranged in an axial direction, a liquid outlet is provided between the annular body and the base body, a center through-hole of the annular body is in communication with the liquid outlet, the center through-hole is coaxial with the medium inlet, the blades are located between the annular body and the base body, the annular body is internally encapsulated with the magnet steel assembly, and the dynamic magnetic ring and the magnetic member are encapsulated inside the base body.

In some embodiments, the base body is provided with an annular encapsulation cavity; the dynamic magnetic ring is sleeved in an inner wall of the annular encapsulation cavity; the magnetic member encapsulated in the base body is located on a periphery of the dynamic magnetic ring; and in a radial direction, an axial height of a middle area of the annular encapsulation cavity is greater than an axial height of an edge area.

In some embodiments, the magnetic levitation centrifugal pump further includes a base and a cover body, wherein the cover body is provided with a cylinder with an opening at one end and a flow guide cone connected with the other end of the cylinder, the opening of the cylinder is circumferentially sealed and fastened to the base, the static magnetic ring is fixed to the base by a threaded member and located inside the cylinder, the base is in a threaded and sealed connection with the volute and is coaxial with the medium inlet, and the flow guide cone passes through a central hole of the annular encapsulation cavity and protrudes towards the medium inlet.

In some embodiments, a first auxiliary channel is formed between an outer peripheral surface and an outer end face of the annular body and a corresponding inner wall of the volute; a second auxiliary channel is formed between both an outer peripheral surface and an outer end face of the annular encapsulation cavity and the corresponding inner wall of the volute, as well as between an inner peripheral wall of the annular encapsulation cavity and the cover body; and both an outer end face of the annular body and an outer end face of the base body have a predetermined included angle with a horizontal plane, and from outside to inside, a distance between the outer end face and the horizontal plane increases.

In some embodiments, an outer end faces of the annular body and the base body are both provided with a plurality of protrusions; each of the plurality of the protrusions extends from an inner edge side to an outer edge side, and the protrusions have a predetermined included angle with a radial direction; wherein the closer to the inner edge side, the smaller a distance between adjacent protrusions, or the closer to the inner edge, the lower a height of each of the plurality of protrusions;

In some embodiments, the rotor is a centrifugal fully-enclosed rotor structure, the blades are backward-curved blades, the magnet steel assembly and the magnetic member are respectively provided on a first end portion and a second end portion of the rotor body, and the blades are located between the magnet steel assembly and the magnetic member;
or/and the magnetic member includes a magnetic ring.

In the present invention, the rotational driving and axial position control of the rotor are completely independent, and are respectively located on two sides of the rotor body; and the control logic is relatively simple. Furthermore, in the present invention, by the magnetic interaction between the dynamic magnetic ring and the static magnetic ring, the full levitation operation of the rotor is able to be achieved, such that there is no mechanical contact between the rotor and the volute (equivalent to the stator), thereby reducing heat generation and wear, and maximally reducing the possibility of thrombus generation and crushing damage to blood cells; and radial levitation limitation of the rotor is able to be achieved by the dynamic magnetic ring and the static magnetic ring.

### Brief Description of the Drawings

Fig. 1 is a three-dimensional structural schematic view of a magnetic levitation centrifugal pump according to an embodiment of the present invention;
Fig. 2 is a cross-sectional three-dimensional view of the magnetic levitation centrifugal pump;
Fig. 3 is a cross-sectional structural schematic view of Fig. 1;
Fig. 4 is a structural schematic view of a rotor according to an embodiment of the present invention;
Fig. 5 is a cross-sectional schematic view of the rotor shown in Fig. 4 from another perspective;
Fig. 6 is a structural schematic view of a rotor body according to an embodiment of the present invention;
Fig. 7 is a schematic view of a rotor according to another embodiment of the present invention;
Fig. 8 is a schematic view of a rotor according to yet another embodiment of the present invention.

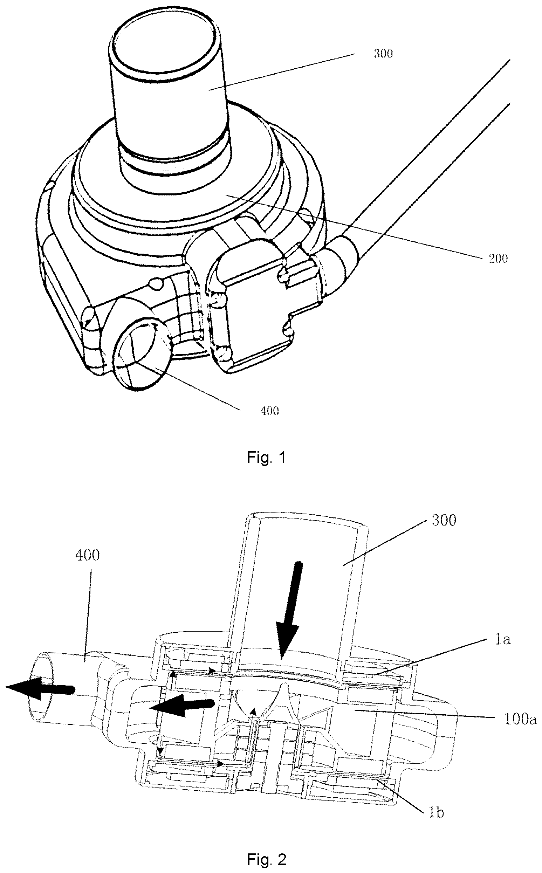

In Figs. 1 to 8:
100 rotor; 11 base body; 111 lower cover plate; 112 annular sealed cavity; 113 inner peripheral wall; 12 annular body; 121 upper cover plate; 1211 outer end face; 1212 protrusion; 13 blade; 14 magnet steel assembly; 15 magnetic component; 16 dynamic magnetic ring; 17 magnetic member;
200 volute; 201 first volute; 202 second volute; 203 first annular housing; 204 second annular housing; 21 driving coil assembly; 211 driving coil; 212 working iron core; 22 static magnetic ring; 23 base; 24 cover body; 241 cylinder; 242 flow guide cone; 26 position sensor; 27 magnetic levitation coil assembly; 271 magnetic levitation coil; 272 magnetic levitation iron core;
300 inlet pipe;
400 outlet pipe;
1a first auxiliary channel; 1b second auxiliary channel; 100a liquid outlet.

### Detailed Description of the Embodiments

In the description of the present invention, it should be noted that, orientation or position relationships indicated by terms such as "left", "right", "upper", "lower", "inside", "outside" and the like are orientation or position relationships based on the accompanying drawings, which are only used for simplifying the technical description, and do not indicate or imply that the device or element referred to must have a specific orientation, a specific orientation configuration and operation, and therefore cannot understand the limitation to the present invention. In addition, terms such as "first", "second" and the like are only used to facilitate description of two or more structures or components whose structures and/or functions are the same or similar, and do not represent a particular limitation to order and/or importance.

Without loss of generality, the technical solutions and technical effects are introduced herein by taking the invention of the magnetic levitation centrifugal pump to heart blood pumping as an example. A person skilled in the art should understand that although the magnetic levitation centrifugal pump of the present invention is a technical solution proposed on the basis of research on blood pumps, the magnetic levitation centrifugal pump of the present invention is not limited to being applied to heart blood pumping, but its invention in other fields is still within the scope of protection of the present invention.

To make persons skilled in the art better understand the technical solutions of the present invention, the present invention is further described in detail with reference to the accompanying drawings and specific embodiments.

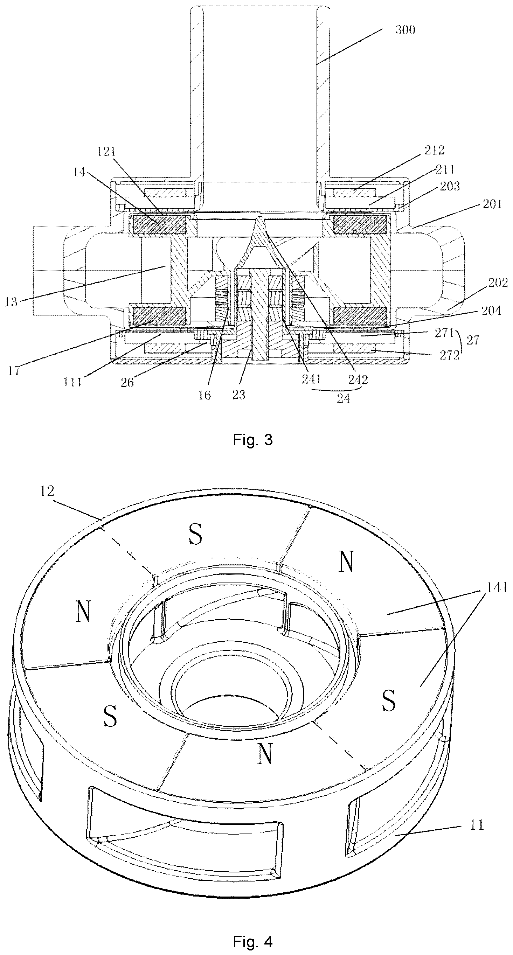

Please refer to Fig. 1 to Fig. 3, Fig. 1 is a three-dimensional structural schematic view of a magnetic levitation centrifugal pump according to an embodiment of the present invention; Fig. 2 is a cross-sectional three-dimensional view of the magnetic levitation centrifugal pump; and Fig. 3 is a cross-sectional structural schematic view of Fig. 1.

The present invention provides a magnetic levitation centrifugal pump, including a volute, a static magnetic ring and a rotor, wherein the volute is provided with a levitation cavity, a medium inlet and a medium outlet, and the rotor is located inside the levitation cavity.

The volute may include a first volute 201 and a second volute 202, wherein the first volute 201 and the second volute 202 enclose a mounting space for the rotor, and the first volute 201 and the second volute 202 are detachably mounted so as to facilitate the mounting and maintenance of components such as the rotor. The medium inlet may be provided on the first volute 201, and the medium outlet may be jointly enclosed by corresponding structures on the first volute 201 and the second volute 202. An inlet pipe 300 is mounted at the medium inlet, an outlet pipe 400 is mounted at the medium outlet, and the first volute 201, the second volute 202, the inlet pipe 300 and the outlet pipe 400 may all be made of a titanium alloy material.

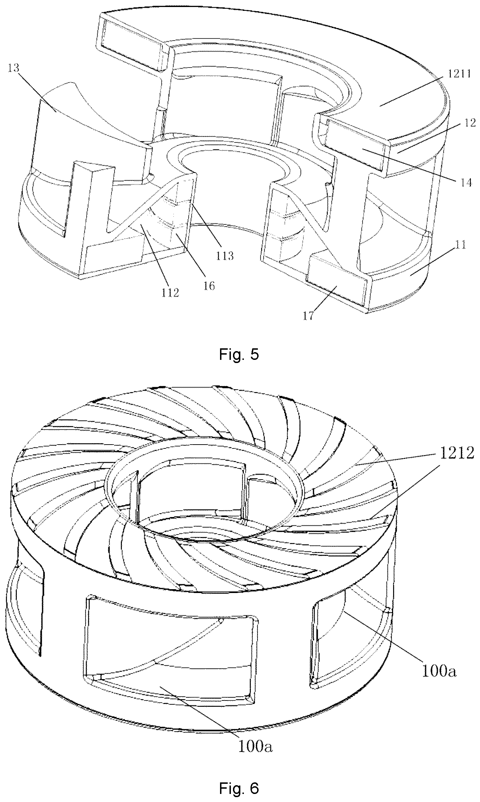

Please further refer to Figs. 4 and 5, Fig. 4 is a structural schematic view of a rotor according to an embodiment of the present invention, and Fig. 5 is a schematic view the rotor shown in Fig. 4 from another perspective.

The rotor in the present invention includes a rotor body, blades 13, a dynamic magnetic ring 16, a magnetic member 17 and a magnet steel assembly 14.

The rotor body mainly provides a mounting base for the mounting of other components of the rotor and is fitted with the volute. A specific structure of the rotor body will be described in detail hereinafter. The dynamic magnetic ring 16, blades 13, magnetic member 17 and magnet steel assembly 14 are all mounted on the rotor body. A number of the blades 13 may be two or more, that is, the number of the blades is at least two, and the blades are distributed circumferentially. The blades 13 may be backward-curved blades. The backward-curved blades achieve optimized fluid efficiency, shear force and streamline distribution, and under same requirements for output flow and pressure, diameters of the rotor and the volute are able to be smaller, and the requirements for a motor speed and a torque are able to be lower, such that the volume of the volute, rotor and motor is able to be reduced, achieving pump miniaturization under same output capacity conditions, and minimizing a possibility of hemolysis and thrombosis to the greatest extent.

The number of the blades 13 may be determined according to a specific pump volume, and may generally be 3 to 7, for example, in a specific example, the number of the blades is 5.

Certainly, the blades 13 may also be equal-thickness blades or straight blades as long as they are able to meet the usage requirements.

The static magnetic ring 22 is mounted on the volute 200, and the static magnetic ring 22 is coaxial and nested with the dynamic magnetic ring 16 to limit a radial position of the rotor 100 relative to the volute 200. Both the static magnetic ring 22 and the dynamic magnetic ring 16 may include two or more annular magnets arranged in an axial direction. Fig. 3 shows a specific example in which both the static magnetic ring 22 and the dynamic magnetic ring 16 have three annular magnets, and the dynamic magnetic ring 16 is nested around a periphery of the static magnetic ring 22. Certainly, a numbers of the annular magnets in the static magnetic ring 22 and the dynamic magnetic ring 16 are not limited to those described herein, and may also be other values.

During operation, a principle by which the dynamic magnetic ring 16 and the static magnetic ring 22 limit the radial position of the rotor relative to the volute 200 is as follows: a radial levitation of the rotor is achieved by a repulsive force between the dynamic magnetic ring and the static magnetic ring; as stated above, the rotor body is provided with a group of dynamic magnetic ring, while the volute is provided with a group of static magnetic ring; and the dynamic magnetic ring and the static magnetic ring together form a permanent magnet radial levitation bearing. An axial position of the static magnetic ring is able to be adjusted by precise threads between the base 23 and the volute 200. In an ideal condition, the position of the static magnetic ring 22 is adjusted, such that when the rotor body is axially levitated in a middle of the volute levitation cavity, the axial positions of the static magnetic ring 22 and the dynamic magnetic ring 16 are exactly aligned. In this case, a radial stiffness of the permanent magnet radial levitation bearing formed by the dynamic magnetic ring 16 and the static magnetic ring 22 is the maximum, while an axial force is zero.

In the present invention, the magnet steel assembly 14 is fixed on a first end portion of the rotor body. The magnet steel assembly 14 includes N first magnet steels 141 arranged in a circumferential direction, and magnetic poles of all the first magnet steels 141 are arranged in an alternating manner. Please refer to Fig. 4, the first magnet steels in the magnet steel assembly are arranged alternately with N poles and S poles to form a circle. The magnet steels in the magnet steel assembly 14 may be encapsulated inside the rotor body. In an example, the first magnet steels 141 are able to be closely attached to each other to form a magnet ring with a full pole arc, such that the disc-shaped motor formed by this configuration and the driving coil assembly 21 mounted on the volute 200 is able to achieve a relatively high motor efficiency.

Certainly, the magnet steel assembly 14 is able to further include transverse conductive magnet steels 18. The magnetic conductive steels 18 are located between the first magnet steels 141, that is, an equal number of mutually repelling transverse conductive magnet steels 18 are arranged between the first magnet steels 141 with alternating magnetic poles, for example, a Halbach magnet steel array consisting of 10 groups of alternating first magnet steels and conductive magnet steels (4-16 even groups are possible, and 10 groups are an optional solution). Such a magnet steel array is able to achieve an effect of magnetic focusing, and improves the magnetic density in an air gap of the motor under the same volume of the magnet steels, thereby further improving the efficiency of the motor.

Certainly, the configuration of the magnet steel assembly 14 is not limited to the manner described herein, and may also be implemented in other manners, as long as it is able to achieve the functions described herein.

Accordingly, a first end portion of the volute 200 corresponding to the magnet steel assembly 14 mounted on the rotor is encapsulated with a driving coil assembly 21, wherein the driving coil assembly 21 may include a driving coil 211 and a working iron core 212. During operation, a current is introduced into the driving coil 211 to generate a magnetic field, the working iron core 212 serves a function of amplifying the magnetic field generated by the driving coil 211, and a rotating force will be generated by the first magnet steels 141 with alternately arranged magnetic poles of the magnet steel assembly 14 mounted on the rotor body, thereby driving the rotor body to rotate. The driving coil assembly 21 and the magnet steel assembly 14 mounted inside the rotor body form a disc-shaped motor.

When a magnetic levitation coil assembly 27 is energized, the magnetic member 17 and the magnetic levitation coil assembly 27 generate an axial force for controlling the axial position of the rotor body. By adjusting the current direction of the magnetic levitation coil assembly 27, the force direction between the magnetic levitation coil assembly 271 and the magnetic member 17 is able to be changed. The magnetic levitation coil assembly 27 may include a magnetic levitation coil 271 and a magnetic levitation iron core 272. The axial position of the rotor body is able to be determined by a position sensor.

In this embodiment, the rotational driving and axial position control of the rotor are completely independent, and are respectively located on two sides of the rotor body, and the control logic is relatively simple. Furthermore, in the present invention, by the magnetic interaction between the dynamic magnetic ring 16 and the static magnetic ring 22, full levitation operation of the rotor is able to be achieved, such that there is no mechanical contact between the rotor 100 and the volute 200 (equivalent to the stator), thereby reducing heat generation and wear, and maximally reducing the possibility of thrombus generation and crushing damage to blood cells; and a radial levitation limitation of the rotor 100 is able to be achieved by the dynamic magnetic ring and the static magnetic ring.

In this specific example, an inner cavity of the volute 200 is provided with a first annular housing 203 and a second annular housing 204; the first annular housing 203 and the second annular housing 204 are respectively located on a first end portion and a second end portion of the volute; the first annular housing 203 and the volute enclose a first sealed cavity for encapsulating the driving coil assembly; the second annular housing and the volute enclose a second sealed cavity for encapsulating the magnetic levitation coil assembly; the levitation cavity is formed between the first annular housing and the second annular housing; the first annular housing 203 and the second annular housing 204 are both of a ceramic structure; the driving coil assembly 21 is attached to the first annular housing 203; and the magnetic levitation coil 271 is attached to the second annular housing 204.

The annular housings may be fixed to the volute by adhesive or other means.

The ceramic material has good compatibility with blood, and the ceramic material is very hard and insulating, such that a wall thickness of the annular housings is able to be relatively thin, and the driving coil is able to be placed attached to the inner wall, thereby greatly reducing the air gap between the driving coil and the first magnet steels and completely eliminating eddy current losses. The first magnet steels are able to be arranged in a Halbach array, improving the efficiency of the motor, and realizing the miniaturization of a blood pump while keeping the maximum output capability unchanged. Due to the insulation property of ceramic, a risk of leakage current generated by the driving coil to the blood flowing in the volute is able to be reduced to the maximum extent, eliminating the possibility of external electric field interference in the operation of the disc-shaped motor formed by the driving coil assembly and the first magnet steel assembly. For example, when a patient undergoes electric shock/electrical cardioversion/electric scalpel cutting treatment, the magnetic levitation centrifugal pump still operates normally.

In an example, the rotor body includes an annular body 12 and a base body 11, the annular body and the base body are fixedly arranged in the axial direction, the blades are located between the annular body 12 and the base body 11, a liquid outlet is provided between the annular body 12 and the base body 11, the liquid outlet may be located between two blades, a central through-hole of the annular body 12 is in communication with the liquid outlet 100a, the annular body 12 and the base body 11 are both encapsulated with magnet steel components, and the dynamic magnetic ring 16 is packaged inside the base body 11. The annular body 12 and the base body are fixedly arranged in the axial direction, a liquid outlet is provided between the annular body 12 and the base body 11, a central through-hole of the annular body 12 is in communication with the liquid outlet, and the central through-hole of the annular body 12 is coaxial with the medium inlet. There may be a plurality of liquid outlets 100a, which are uniformly arranged in the circumferential direction; and the specific number may be determined according to a specific product, which is not limited herein.

In some embodiments, the base body is provided with an annular encapsulation cavity, the dynamic magnetic ring is sleeved in an inner wall of the annular encapsulation cavity, the magnetic member encapsulated in the base body is located on a periphery of the dynamic magnetic ring, and in a radial direction, an axial height of a middle area of the annular encapsulation cavity is greater than an axial height of an edge area.

In this way, an occupation of the internal fluid space by the encapsulation cavity is able to be reduced as far as possible, which is beneficial to a compact structure.

To facilitate assembly, the annular encapsulation cavity may be formed in the following manner: an annular groove is provided on the base body, and a lower cover plate 111 covers a groove opening of the annular groove to form a sealed cavity. Similarly, the sealed cavity for mounting the magnet steel assembly on the annular body may also be formed by providing an annular groove to cooperate an upper cover plate 121 for sealing.

The rotor in the present invention may be a centrifugal fully enclosed rotor. When the centrifugal pump operates, a large amount of blood flows into the centrifugal pump through an inflow channel, accelerates by the centrifugal blades of the rotor, and flows out of an outflow channel, thereby injecting blood into an aorta to provide pressure and flow for the blood circulation throughout the body. The centrifugal blades of the rotor may have a hollow structure.

In the described embodiments, the static magnetic ring may be mounted inside the volute in the following manner.

In an example, the magnetic levitation centrifugal pump may further include a base and a cover body 24 snap-fitted to the base 23, the cover body 24 is sealed and fixed to the base, the static magnetic ring is fixed to the base by a threaded member, and the threaded member may be component such as a screw, a bolt, a rod or the like. Furthermore, the static magnetic ring is mounted in a mounting space formed by the cover body and the base, a second end portion of the volute is provided with a mounting through-hole, the mounting through-hole is coaxial with the medium inlet, and the base 23 is in a threaded and sealed connection with the mounting through-hole. The cover body 24 includes a cylinder 241 provided with an opening at one end and a flow guide cone 242 connected with the other end of the cylinder, the static magnetic ring 22 is located inside the cylinder 241, and the flow guide cone 242 passes through a central hole of the base body 11 and protrudes towards the medium inlet. The closer the flow guide cone 242 is to the medium inlet, the smaller the radial size is, such that the fluid at the medium inlet of the volute is able to flow evenly in the circumferential direction under the flow diversion of the flow guide cone, and then evenly enters between the blades.

In the described embodiment, the base is in threaded connection with the volute, such that the axial position of the base relative to the volute is able to be precisely adjusted so as to achieve precisely matching with the dynamic magnetic ring on the rotor.

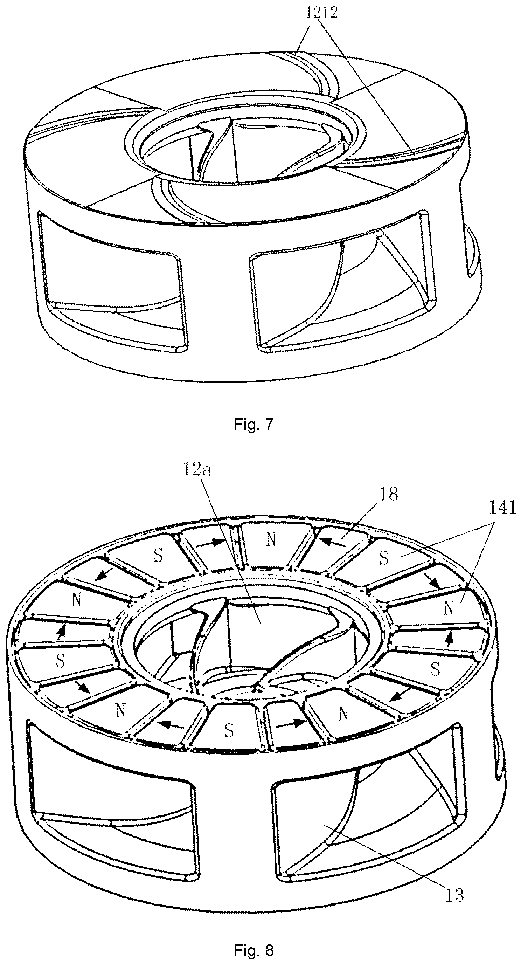

Please refer to Figs. 6 and 7, in the described embodiments, an outer end face of the annular body 12 and an outer end face of the base body are both provided with a plurality of protrusions 1212 (only the protrusions of the outer end face of the annular body are shown in the drawings), the protrusions extend from an inner edge side to an outer edge side, the protrusions 1212 and the radial direction have a predetermined angle, a distance between adjacent protrusions decreases as they get closer to the inner edge side, and the protrusions 1212 form an internal spiral structure. In this way, a hydrodynamic fluid bearing is formed between the outer end face of the annular body and the first annular housing and between the outer end face of the base body and the second annular housing. When the rotor is greatly interfered in the axial direction and one end is close to the annular housing at the side, the hydrodynamic fluid bearing is able to provide an additional restoring force towards the center, thereby improving the stability of an impeller in the axial direction.

Certainly, a height of the protrusions 1212 decreases as they get closer to the inner edge, and this is able to achieve the same technical effect, as shown in Fig. 7.

In a specific embodiment, a first auxiliary channel is formed between an outer peripheral surface and the outer end face of the annular body and a corresponding inner wall of the volute; and a second auxiliary channel is formed between an outer peripheral surface and the outer end face of the annular encapsulation cavity and the corresponding inner wall of the volute, and between an inner peripheral wall 113 of the annular encapsulation cavity and the cover body 24.

During operation, the rotor levitates and rotates at a high speed in the middle of the volute 200; the blades between the annular body 12 and the base body 11 and the inner wall of the volute 200 form a main flow channel of the pump; and blood flows in from the medium inlet, enters the main flow channel between the blades through the central through-hole of the annular body, accelerates by the rotation of the blades, enters the main flow channel inside the volute, and flows out of the volute through the medium outlet.

At the same time, a small portion of blood entering the main flow channel inside the volute 200 reflows back to the inlet of the rotor body through the first auxiliary channel and the second auxiliary channel respectively, reenters between the blades and flows into the volute after being accelerated. By the described design, all the channels through which blood flows inside the centrifugal pump are unidirectional, and there is no stagnant or backflow area, thereby minimizing the possibility of thrombus formation.

Meanwhile, as described above, as the annular housings opposite to the end faces of the annular body and the base body are made of a ceramic material, the surfaces of the annular housings are hard and smooth, such that when the rotor accidentally contacts the annular housings, the possibility of damage to the surfaces of the annular housings is reduced. Due to the higher efficiency of the motor, under the same condition, a thickness of the first magnet steels in the rotor is able to be reduce, and a thickness of an outer end wall (the cover plate) of the annular body 12 and base body 11 is also correspondingly reduced, thereby reducing a length of a return flow channel in the volute.

Furthermore, the outer end face of the annular body and the outer end face of the base body both have a predetermined angle with a horizontal plane, and a distance between the outer end faces and the horizontal plane increases from outside to inside, that is, the outer end faces of the annular body and base body are recessed inwards, and the upper and lower outer end faces of the rotor are tapered inwards. Therefore, for the auxiliary channel formed between the outer end faces and the inner wall of the volute, the outer gap is smaller while the inner gap is larger. In this way, when the blood passes through the smaller outer gap area, the shear force is relatively high but the flow rate is high, resulting in a short passing time; and when the blood passes through the larger inner gap area, the relative flow rate is low, but the shear force is also low, thereby reducing the possibility of hemolysis and thrombus generation to the maximum extent. In this way, the gap of the auxiliary channel is relatively reduced, reducing the reflow loss of the centrifugal pump, and improving the fluid efficiency. Under the same output flow/pressure requirements of the centrifugal pump, the rotor and volute are able to have smaller diameters, and the requirements for motor speed and torque are able to be lower, reducing the volume of the volute/rotor, and realizing the miniaturization of the centrifugal pump while maintaining the same output capacity.

The position sensor may be a Hall sensor or an eddy current sensor, as long as it is able to detect the axial position of the rotor body.

The magnetic levitation centrifugal pump provided by the present invention is described in detail above. The principle and embodiments of the present disclosure are described herein through specific examples, and the description of the embodiments is only used to help understand the method and core idea of the present disclosure. It should be noted that, a person of ordinary skill in the art may further make improvements and modifications to the present invention without departing from the principle of the present invention, and these improvements and modifications also belong to the scope of protection of the claims of the present invention.

## Claims

1. A magnetic levitation centrifugal pump, comprising a volute, a static magnetic ring and a rotor;
the volute is provided with a levitation cavity, a medium inlet and a medium outlet, the rotor is located inside the levitation cavity, and the static magnetic ring is fixed to the volute;
the rotor comprises a rotor body, a dynamic magnetic ring positioned on the rotor body, and at least two blades; the dynamic magnetic ring is coaxial and nested with the static magnetic ring to limit a radial position of the rotor body relative to the volute;
the rotor body is fixedly provided with a magnet steel assembly, the magnet steel assembly comprises N first magnet steels arranged in a circumferential direction, and magnetic poles of all the first magnet steels are arranged in an alternating manner; the rotor body is further fixedly provided with a magnetic member, and the magnetic member comprises at least one of a magnetic ring and an iron core;
the volute is encapsulated with a driving coil assembly arranged opposite the magnet steel assembly, and the driving coil assembly cooperates with the magnet steel assembly to enable the rotor body to rotate in the circumferential direction; and the volute is fixedly provided with a magnetic levitation coil assembly, and when the magnetic levitation coil assembly is energized, the magnetic member and the magnetic levitation coil assembly generate an axial force.

2. The magnetic levitation centrifugal pump according to claim 1, further comprising a position sensor, configured to detect an axial position of the rotor body;
or/and adjacent first magnet steels are closely attached, or the magnet steel assembly further comprises transverse magnetic conductive magnet steels, each of the magnetic conductive magnet steels is located between two of the first magnet steels, and all the magnetic conductive steels and all the first magnet steels form a Halbach magnet steel array.

3. The magnetic levitation centrifugal pump according to claim 1, wherein the magnet steel assembly and the magnetic member are respectively provided on a first end portion and a second end portion of the rotor body, and the driving coil assembly and the magnetic levitation coil assembly are respectively provided on a first end portion and a second end portion of the volute.

4. The magnetic levitation centrifugal pump according to any one of claims 1 to 3, wherein an inner cavity of the volute is provided with a first annular housing and a second annular housing; the first annular housing and the second annular housing are respectively located on a first end portion and a second end portion of the volute; the first annular housing and the volute enclose a first sealed cavity for encapsulating the driving coil assembly; the second annular housing and the volute enclose a second sealed cavity for encapsulating the magnetic levitation coil assembly; the levitation cavity is formed between the first annular housing and the second annular housing; the first annular housing and the second annular housing are both of a ceramic structure; the driving coil assembly is attached to the first annular housing; and the magnetic levitation coil is attached to the second annular housing.

5. The magnetic levitation centrifugal pump according to any one of claims 1 to 3, wherein the rotor body comprises an annular body and a base body, wherein the annular body and the base body are fixedly arranged in an axial direction, a liquid outlet is provided between the annular body and the base body, a center through-hole of the annular body is in communication with the liquid outlet, the central through-hole is coaxial with the medium inlet, the blades are located between the annular body and the base body, the annular body is internally encapsulated with the magnet steel assembly, and the dynamic magnetic ring and the magnetic member are encapsulated inside the base body.

6. The magnetic levitation centrifugal pump according to claim 5, wherein the base body is provided with an annular encapsulation cavity; the dynamic magnetic ring is sleeved in an inner wall of the annular encapsulation cavity; the magnetic member encapsulated in the base body is located on a periphery of the dynamic magnetic ring; and in a radial direction, an axial height of a middle area of the annular encapsulation cavity is greater than an axial height of an edge area.

7. The magnetic levitation centrifugal pump according to claim 6, further comprising a base and a cover body, wherein the cover body is provided with a cylinder with an opening at one end and a flow guide cone connected with the other end of the cylinder, the opening of the cylinder is circumferentially sealed and fastened to the base, the static magnetic ring is fixed to the base by a threaded member and located inside the cylinder, the base is in a threaded and sealed connection with the volute and is coaxial with the medium inlet, and the flow guide cone passes through a central hole of the annular encapsulation cavity and protrudes towards the medium inlet.

8. The magnetic levitation centrifugal pump according to claim 7, wherein a first auxiliary channel is formed between an outer peripheral surface and an outer end face of the annular body and a corresponding inner wall of the volute; a second auxiliary channel is formed between both an outer peripheral surface and an outer end face of the annular encapsulation cavity and the corresponding inner wall of the volute, as well as between an inner peripheral wall of the annular encapsulation cavity and the cover body; and both an outer end face of the annular body and an outer end face of the base body have a predetermined included angle with a horizontal plane, and from outside to inside, a distance between the outer end face and the horizontal plane increases.

9. The magnetic levitation centrifugal pump according to claim 5, wherein an outer end faces of the annular body and the base body are both provided with a plurality of protrusions; each of the plurality of the protrusions extends from an inner edge side to an outer edge side, and the protrusions have a predetermined included angle with a radial direction; wherein the closer to the inner edge side, the smaller a distance between adjacent protrusions, or the closer to the inner edge, the lower a height of each of the plurality of protrusions.

10. The magnetic levitation centrifugal pump according to claim 1 or 2, wherein the rotor is a centrifugal fully-enclosed rotor structure, the blades are backward-curved blades, the magnet steel assembly and the magnetic member are respectively provided on a first end portion and a second end portion of the rotor body, and the blades are located between the magnet steel assembly and the magnetic member;
or/and, the magnetic member comprises a magnetic ring.
